# EUROPEAN PATENT SPECIFICATION

(11) **EP 1 159 966 B1**
(45) Date of publication and mention of the grant of the patent: **23.10.2002**
(21) Application number: 00907415.4
(22) Date of filing: 22.02.2000
(51) Int. Cl.: A61K 35/50, A61P 17/06

(54) **COMPOSITION FOR THE TREATMENT OF PSORIASIS**
ZUSAMMENSETZUNG ZUR BEHANDLUNG VON PSORIASIS
COMPOSITION POUR LE TRAITEMENT DU PSORIASIS

(30) Priority: 22.02.1999 CU 1699
(43) Date of publication of application: 05.12.2001
(73) Proprietor: Centro de Histoterapia Placentaria, La Lisa, Ciudad de la Habana 17100 (CA)
(72) Inventor: MIYARES CAO, Carlos Manuel, Playa, Ciudad de la Habana 11300 (CU)
(74) Representative: Prins, Adrianus Willem
(86) International application number: CU0000001
(87) International publication number: WO00050047

(56) References cited:
- DATABASE EPODOC [Online] EUROPEAN PATENT OFFICE, THE HAGUE, NL; XP002901074 & WO 97 48405 A (BELYAEV JURY EVGENIEVICH) 24 December 1997 (1997-12-24)
- DATABASE WPI Section Ch, Week 199605 Derwent Publications Ltd., London, GB; Class B04, AN 1996-049410 XP002901075 & WO 95 34310 A (LENIMS STOCK CO), 21 December 1995 (1995-12-21)
- DATABASE WPI Section Ch, Week 198433 Derwent Publications Ltd., London, GB; Class B04, AN 1984-206318 XP002901076 & SU 1 061 818 A (SKIN VENEROLOGY RES), 23 December 1983 (1983-12-23)
- DATABASE WPI Section Ch, Week 199721 Derwent Publications Ltd., London, GB; Class B04, AN 1997-227094 XP002901077 & CN 1 098 310 A (LI Q), 8 February 1995 (1995-02-08)
- DATABASE STN INTERNATION. [Online] NOVIKOVA Z I: "Effect of placenta suspension on the immunologic indicators and dynamics of skin changes in patients with palmar and plantar pustulous psoriasis" retrieved from MEDLINE, accession no. 86099200 XP002901078 & VESTNIK DERMATOLOGII I VENEROLOGII, no. 10, October 1985 (1985-10), pages 46-48, ISSN: X9U-0042-4609

## Description

### Technical sector

The present invention is related with the branch of the Human Medicine, and with the Dermatology, and in particular with a composition obtained starting from the human placenta with activity in the regulation of the speed of regeneration of the skin, and therefore useful in the treatment of the psoriasis, as well as with the procedure for obtaining said composition.

### Prior Art

The psoriasis is not a recently discovered illness. It was classified as the leprosy of the Greeks and as it seems, it existed in Mexico before the conquest (Daune, M.; FSCHS, P.P. La Recherche (1980), 11: 116-1066; International Journal of Dermatology. Vol. 36, No. 4, April 1977). This illness affects all the races and in all the latitudes and it can be presented in a family in the course of several generations (Int. Journal of Dermatology. Vol. 36, No.4, April 1977; Lerner, A. B., et al. Invest. Dermatol. 1975. 20 (2): 299-314, 1953).

The beginning of the illness often it is associated with superficial damages of the skin as burns and scratches. Other factors like low humidity, infections, stress or drugs can precipitate or to exacerbate the psoriasis.

The physical trauma maybe incites an angiogenic factor of change in the vascular fact and the epidermatopoyesis and the neutrophile chimiotasis, and the called tissular psoriatic reaction (Moroni, P., et al. Derm. Beruf. Umwelt, 36 (5) pág. 163-164, 1988).

The climatic factors such as cold have been reported as adverse effects to the psoriasis. In some studies carried out it was bigger the frequency psoriasis in Africa where the climate is dry and the humidity is low (Faber, E. N., et al. Natural Epidemiology. History and genetic psoriasis. Second Ed. Page 231. Edit. Marcel Depper, New York, 1991).

It is very well known that the stress can break out or to exacerbate the psoriasis, although the exact mechanism for that is unknown, however studies based on psiconeuroimmunology can explain it (Moroni, P., et al. Derm. Beruf. Umwelt, 36 (5) Pag. 163-164, 1988).

As a result of the specialized treatment, the lesions can disappear but the relapses are almost sure and tendencies exist to that its effectiveness gradually is lost in each treatment (Lerner, A. B., et al. Invest. Dermatol. 1953, 20 (2): 299-314, 1953; Roenigk, H. H. Jr., et al. Arch. Dermatol. 113(12): 1667-1670, 1977; Frappazz, A., et al. Env. J. Dermatol. 1993, 3: pág. 351-354; Mozzanica, N., et al. Acta Dermatol. Venereol. 1994, 186: pág. 171-172; Ortonne, J. P.New Dermaytol. 1994, 13: pág. 746-751; Panizonni R. Schweizerische Rundschav fur Medizin Praxis 1995, 84: pág. 649-653; Kirsner, R. S. Am. Fam. Phys. 1995, 52: pages 237-240).

It is known of the state of the art the use of diverse preparations like pitches in petrolatum or alcoholic solutions for the treatment of the psoriasis.

From last century the antialine is used and it has occupied an important roll in the treatment of the illness, but it has the inconvenience of causing irritations of the skin.

In 1925 the ultraviolet radiation was introduced that has continued being used, alone or combined with other medications, but it has its adverse collateral effects.

The psoralens are also used from in the past in spite of their proven toxic effect.

The corticosteroids used in topical way in general have been useful in selected cases, but the tendency to rebound limits their use.

Recently it has been begun to use, but with scarce successes, the retinoids, some anti-metabolites like metotrexate, thioguamne and others.

From the century III it is reported from China the use of the placenta as biological stimulant.

Starting from 1945 the Soviet researcher Filatov developed and consolidated a method to isolate substances with healing properties starting from the animal tissues and vegetables conserved in cold to which he denominated histotherapy. Within of it the placenta occupies an important place (Filatov, V. Ed. Medicina Medguis. URSS, 1945).

Orechkin in 1963, based on studies carried out with 300 sick persons of cutaneous affections, reaches the conclusion that under the influence of the histotherapy the cure of 25% of the cases of an improvement more or less apparent can be obtained in the 38,5% of the same. (Orechkin, Material E. of the Scientific Conference dedicated to the 30 anniversary of V. Filatov's histotherapy method. Kiev: 12, 1963).

The mechanism of therapeutic action of the biogenic stimulators of the psoriasis was ignored. Makarov and Romani pointed out that under its effects the formation of suprarenal corticoids it is intensified and using the experimental theory with the paws of the rats demonstrated that they also possesses anti-inflammatory action therefore being able to behave in a same way that these hormones (Makarov and Romani. Comp. Rend. Soc. Biol. 1951, 1966: 4-626).

The therapeutic means for the treatment of this illness have varied according to their localization, graveness, duration, previous treatments and the patient's age.

Of the many resources with which the medicine has to combat the psoriasis, the placental histotherapy is one of the most effective and however one of the less well known.

### Disclosure of the invention.

The present invention relates to compositions as defined in the claims.

The present invention is related with an extract obtained starting from the human placenta, which is incorporated in a hydrosoluble gel, and that it is constituted in a medication of having proven effectiveness for the treatment of the psoriasis.

Although the route of administration preferably for these stimulators is the parenteral one, in the present invention it is provided the topical application of this hydrosoluble gel that contains the mentioned active principle.

Therefore, the present invention is related with a hydrosoluble gel that contains as active principle a mixture of unsaturated and poly-unsaturated fatty acids obtained from the human placenta, which intervene in the regulation of the speed of regeneration of the epidermis, after to its transcutaneous absorption from the application place, of here its anti-psoriatic action.

For the obtaining of the active principle useful in the treatment of the psoriasis, the following operations are carried out:
- starting from frozen placentas, previously washed for their separation from the amniotic membrane and the umbilical cord, their cotyledons are separated which are washed again with abundant water;
- the mass of cotyledons is milled to achieve particles of approximately 12 mm of diameter;
- the solid is again washed by means of agitation in water and later on filtered by gauze, being milled again until a particle size between 2 and 6 mm of diameter;
- the obtained mass is agitated during 1-3 hours with 1 to 4 volumes of alcohol per mass of cotyledons, passing later by a gauze;
- the resultant liquid is rotoevaporated and re-dissolved in a mixture of chloroform methanol in the proportion 4:2, staying in maceration during 24 hours more;
- the obtained product is centrifuged to 2000 rpm, the supernatant is rotoevaporated to dryness and finally it is re-dissolved in ethanol in a proportion 1:1.

For the preparation of the formulation of this active principle as a hydrosoluble gel, they are dissolved between 25.5 and 55.8 g of the human placenta extract obtained, in a hydrosoluble excipient constituted by between 35 to 50 g of purified water, between 0.10 to 0.30 g of methyl-paraben and 0.01 to 0.05 of propyl-paraben, to those previous to the addition of the active principle they are added under agitation between 2 to 5 g of carboximetylcellulosa and between 5 to 20 g of glycerin.

To the human placenta extract PE-100 it was determined its composition, finding that it is constituted fundamentally by unsaturated and fatty acids as palmitoleic, and oleic and poly-unsaturated fatty acids as linoleic, arachinoidic and eicosietienoic.

These poly-unsaturated fatty acids are closely related with the therapy of the illness, inhibiting the excessive reproduction of the epidermic cells. Their acceptance for the patients treated evidence the possibility that the composition is broadly used for the proposed application, since so far there is not any report of another product that cures the psoriasis with the innocuousness of the composition of the present invention.

According to clinical reports obtained from the patients, it lacks of noxious secondary effects not only local but also symptomatic.

To determine the effectiveness of the medication, they were carried out open non randomized studies applying as treatment the hydrosoluble gel obtained from the placental extract in the universe of the patient suffering psoriasis.

The Histotherapy Placental Center controlled one of the trials, wherein they were evaluated a total of 400 patients.

To the 2 years of treatment with the medication it was observed a 78% of satisfactory evolution of these patients treaties during this stage.

Another clinical study was carried out with 26 diagnosed patients of psoriasis in a hospital. In the evaluation of the treatment they presented total clearing of the lesions 84,6% of the patients, obtaining satisfactory results in this trial.

In 1990 they began to arrive to Cuba children affected by the nuclear accident happened in Chernobil, old USSR, among them not few of them suffered psoriasis. Of them, 56 patients were studied, patients that were refractory to other medications and, as it is described in the literature, the family cases are in general difficult to try. These patients were treated during 3 months and at the end of the treatment the clinical improvement was achieved in more than 90% of the same ones.

Equally the product of the invention was applied to 100 patients of psoriasis treated in other hospital. Concluded the trial during a period of 6 years, the remission of the illness was achieved in 78% of the cases without registering secondary or symptomatic reactions, neither to take place relapse in 50% of these.

### EXAMPLES:

### Example 1: Description of the process of elaboration of the extract PE-100.

The defrosted placentas are cleaned by removing the umbilical cord and the amniotic membrane, remaining only the cotyledons, which are washed with abundant water. The mass of cotyledons is milled in the mill until achieving particles between 8 and 12 mm of diameter.

The solid moves to the area of reactors by means of rolling tank and the reactor of 1600 L is loaded adding to it between 2-4 volumes of raw water; it is agitated between 2 to 8 minutes and filtered using gauze and milled later up to achieve particles of size between 2 and 6 mm of diameter. Afterwards, the solid is weighted and the 1600 L reactor is loaded with alcohol (1 to 4 volumes of alcohol per Kg of mass of cotyledons. The mixture is agitated from 1 to 3 hours and then it is passed through gauze. The resultant liquid is rotoevaporated and it is re-dissolved in a mixture of chloroform-methanol in a rate of 4:2, keeping it in maceration during 24 hours. Afterwards it is centrifuged to 2000 rpm and the supernatant is rotoevaporated up to dryness and re-dissolved in ethanol in proportion 1:1, being obtained by this way the active principle that is denominated Placental Extract-100 (PE-100).

When the PE-100 is approved by the laboratories of chemical, biological and microbiological controls, it is proceeded to the production of the hydrosoluble gel.

### Example 2: Preparation of a hydrosoluble gel that contains a human placenta extract.

The reactor Olsa is loaded by means of one Bar of vacuum, with the purified water dedicated to the production.

The methyl and propy-lparaben are warmed until their dissolution by means of agitation. The carboximethylcellulosa is mixed with the glycerin during 1 to 3 hours before the beginning of the process to achieve a correct levigate. When the solution is cooled to 30-60°C, the carboxymethylcellulose is incorporated with the glycerin, maintaining constant agitation, incorporating the PE-100 later with soft agitation until having a gel of uniform aspect, taking samples for the control of the process. Once checked for the technological area some samples are taken to carry out the chemical, biological and microbiological controls and by this way to determine the quality of the production batch.

### Example 3: Use of a gel of human placenta extract used through topical way in the treatment of the psoriasis.

The hydrosoluble gel obtained in the Example 2 is used as topical medication in 100 patients of psoriasis belonging to the Service of Dermatology of the General Hospital "Calixto García", in Havana City.

This gel is applied topically with the fingers on the psoriatic lesions, without rubbing, extending it in form of a fine layer.

Its application is carried out with an interval of time of 8 hours, and one of these applications should be accompanied by a previous and later exposition to infrared light during 30 minutes.

Concluded the trial at the end of 2 years, the remission of the illness was achieved in 78% of the cases, without registering local or systemic secondary reactions, neither to take place relapses in 50% of these.

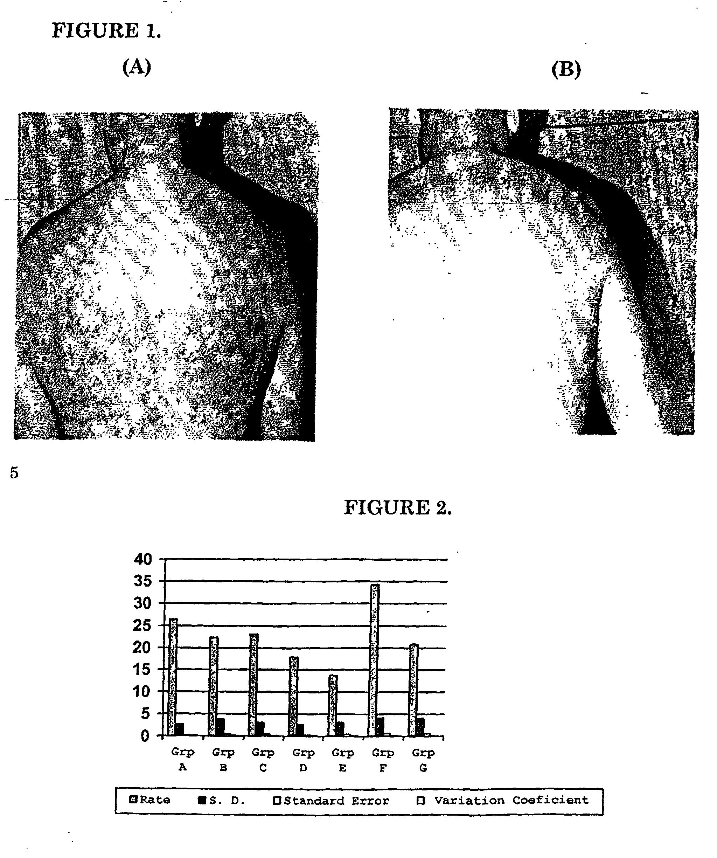

An example of the results achieved in sick persons treated with the composition of the present invention is shown in the Figure 1.

### Example 4: Irritation studies in skin.

The test consisted in the daily dermal application of the anti-psoriatic gel during 90 days, to rats Sprague Dawley (SPF), with the objective to evaluate the risks and/or benefits of the treatment with this substance.

Finished the test, in consideration of the pathology results obtained, in particular those of comparative evaluation of the corporal weight and the weight of the organs and their relationship with the absolute weight, as well as the morphological changes (macro and microscopic), it is concluded that the lesions don't respond to direct effects caused by the substance tested in the formulation used and that the repeated application of the same on the shaved skin was innocuous.

### Example 5: Study of the histological modifications observed in psoriatic plates tried with the hydrosoluble gel of human placenta extract.

They were carried out biopsies in diverse regions of the body affected by psoriatic lesions in the sick persons, before and three months after the daily topical treatment with the gel of human placenta extract, with the purpose of observing the histological modifications produced.

As remission indexes the values were considered the values in micrometer of the high of the interpapilar epidermic crests and the thickness of the horny layer of the total size of the epidermis sample.

The fragments of tissues were processed, not only for the classic technique of double coloration with hematoxilin-eosin, but also for azan.

The numeric data underwent the t-Student statistical analysis of the average comparison by means of a computer program.

The obtained results demonstrated a significant reduction of the corresponding average values, not only of the horny layer but also of the crests, in eight of the studied cases, that which confirms the effectiveness of the treatment statistically.

Only two patients didn't show some improvement, observing a correspondence between the histological finding and the evaluation of their clinical square.

### Example 6: Pharmacological evaluation of diverse anti-psoriatic drugs by means of an experimental model.

The anti-psoriatic activity of the substances to evaluate was carried out according to the experimental model of Spaerman and Jarret (Pharmacological assay for anti-psoriatic drugs. Br. J. Dermatology 92: 581.1975) that bears the topical application of the same on the mouse line.

These substances should diminish the epidermis of the anatomical structure significantly in these animals, if they possess such an effect.

For the trial they were used 35 albino males mice of 20-25g of weight, separated in seven groups of five animals each one, those that were denominated A, B, C, D, E, F and G.

The group A was not treated with any substance to be used as control and to allow the comparison with each one of the substances to study.

The remaining groups were treated in the following ways:
Group B: Desonide ointment.
Group C: Tegrin ointment.
Group D: Gel of human placental extract.
Group E: Betametasone ointment.
Group F: Vitamine A in oil.
Group G: Ointment of pitch.

All the substances were applied topically with the fingers of the operator covered with a rubber thimble, on the tails of the mice starting from one cm of their proximal extreme, covering an extension of 2,5 cm of their longitude in their entire contour.

The time of duration of the topical treatment was of 21 days for all the groups, except the control that didn't receive any treatment.

Lapsed this time the animals were sacrificed to extract them the skin segment that covers the tail in an extension of 3 cm and to carry out the corresponding histological study, for which the skin is lengthwise cut along its surface and it is extended immediately.

The tissue samples are fixed in ethanol to 70% and are included in blocks of paraffin to cut them sagittally in sections of 7 microns of thickness and later on to stain them by means of the habitual technique of Hematoxiline-Eosine.

The epidermis thickness the sample is measured with an ocular micrometer. They were carried out 10 measurements in 10 different regions randomly selected by each line, so that 50 measurements were obtained by each treatment type and for the control

According to the results obtained by means of this study it could be proven that the four substances habitually used in the treatment of the psoriasis (B, C, E and G) significantly diminish the epidermis thickness in the tail of the mouse, regarding the control group, non subjected to any treatment.

It is proven that, on the contrary, the vitamin A eventually used possibly in the treatment of the psoriasis can have a noxious effect in this sense when increasing the epidermis thickness in a statistically significant way.

Jointly it could also be evidenced that the gel of placental extract, object of the invention, reduces significantly as the previous substances the epidermis thickness of the mouse line, what facilitates the inclusion of this formulation inside the therapeutic arsenal for the treatment of this illness.

The results obtained in this study are shown in the Figure 2.

### Example 7: Evaluation of a gel of human placenta with anti-psoriatic action.

A hydrosoluble gel is used with base on Carboxymethylcellulose, Propylene glycol and distilled water which is added an alcoholic extract of human placenta, with the purpose of evaluating the anti-psoriatic action thereof, using it as topical medication in 26 patients carrier of psoriasis. Concluded the trial at the end of 2 years, the remission of the cutaneous lesions was achieved in 22 patients (84,6%), marked improvement in 3 patients for 11,5% and a patient (3,8%) it didn't present change in its lesions.

On the other hand, it was observed that adverse reactions were not presented to the use of the medication.

### Example 8: Clinical and histological studies of a group of children of Chernobyl tried with the anti-psoriatic gel.

The formulation of the invention is used with the purpose of evaluating the anti-psoriatic action of this gel, using it as topical medication in 56 patients of psoriasis affected by this illness after of the Chernobyl nuclear accident.

The results of the treatment were evaluated in the patients of psoriasis during 3 months. When concluding the treatment the anti-psoriatic gel had demonstrated its effectiveness, achieving the clinical and histological improvement in more than 90% of the patients, without being reported in any case undesirable collateral effects.

### Example 9: Evaluation of the use of the formulation of the invention in the Histotherapy Placental Center' s Clinic.

A hydrosoluble gel is used with base on Carboxymethylcellulose, Propylene glycol and distilled water which is added an alcoholic extract of human placenta with bio-stimulant properties with the purpose of evaluating the anti-psoriatic action of the same, using it as topical medication in 400 patients carrying psoriasis that went to the Service of Dermatology of the Clinic belonging to the Histotherapy Placental Center.

The general objective was to know the therapeutic effectiveness of this gel elaborated starting from human placenta in its topical use, in patients with psoriasis. It specific objective was to determine the clinical evolution of the patients treated and to specify presence or not, of possible adverse reactions to the use of the product.

Concluded the study at the end of 2 years, the remission of the cutaneous lesions was achieved in the patients 78% and it was observed that adverse reactions were not presented to the use of the medication.

### Brief Description of the Figures:

**Figures 1:** It represents the evolution of a patient with psoriatic lesions widespread, before (A) and three months after the topical treatment with the gel of placental extract (B).
**Figures 2:** The figure 2 shows the results obtained in the determination of the anti-psoriatic activity of substances to evaluate, according to the experimental pattern of Spaerman and Jarret.

The substances were evaluated in seven animal groups denominated A, B, C, D, E, F and G in correspondence to the treatment to that they were subjected.

The group A it was not treated with any substance (placebo) and the remaining groups were been in the following ways:
Group B: Desonide ointment.
Group C: Tegrin ointment.
Group D: Gel of placental extract human.
Group E: Betametasone ointment.
Group F: Vitamin A in oil.
Group G: Ointment of pitch.

## Claims

1. Composition for topical application for the treatment of the psoriasis containing an alcoholic extract derived from human placenta as active principle, and an appropriate excipient.

2. Composition according to claim 1 wherein this derived alcoholic extract of the human placenta is constituted fundamentally by unsaturated fatty acids such as palmitoleic and oleic acids and poly-unsaturated fatty acids such as linoleic, arachinoidic and eicosietienoic acids.

3. Composition according to claims 1 and 2 wherein for each 100 grams of composition it contains approximately 59 ml of this alcoholic extract in an hydrosoluble excipient.

4. Composition according to claims 1 to 3 wherein it is a hydrosoluble gel.

## Patentansprüche

1. Zusammensetzung zur lokalen Anwendung für die Behandlung der Psoriasis umfassend einen alkoholischen Extrakt, der als aktiver Bestandteil aus der menschlichen Plazenta gewonnen wird, und einen geeigneten Arzneimittelträger.

2. Zusammensetzung nach Anspruch 1, worin der aus der menschlichen Plazenta gewonnene Extrakt grundsätzlich ungesättigte Fettsäuren, wie Palmitolsäure und Ölsäure, und mehrfach ungesättigte Fettsäuren, wie Linolsäure, Arachinoidsäure und Eicosietiensäure, aufweist.

3. Zusammensetzung nach Anspruch 1 oder 2, in der 100 g der Zusammensetzung ungefähr 59ml des alkoholischen Extrakt in einem wasserlöslichen Arzneimittelträger enthalten.

4. Zusammensetzung nach Anspruch 1 bis 3, die durch ein wasserlösliches Gel gebildet wird.

## Revendications

1. Composition pour application topique pour le traitement du psoriasis, contenant un extrait alcoolique dérivé du placenta humain comme principe actif, et un excipient approprié.

2. Composition selon la revendication 1, dans laquelle cet extrait alcoolique provenant du placenta humain est constitué fondamentalement d'acides gras insaturés tels que les acides palmitoléique et oléique et d'acides gras poly-insaturés tels que les acides linoléique, arachinoïdique et eicosiétienoique.

3. Composition selon les revendications 1 et 2, dans laquelle, pour chaque proportion de 100 grammes de composition contient approximativement 59 ml de cet extrait alcoolique dans un excipient hydrosoluble.

4. Composition selon les revendications 1 à 3, dans laquelle elle est un gel hydrosoluble.
